# EUROPEAN PATENT APPLICATION

(11) **EP 3 066 987 A1**
(43) Date of publication of application: **14.09.2016**
(21) Application number: 14860927.4
(22) Date of filing: 14.03.2014
(51) Int. Cl.: A61B 10/02, A61B 6/00

(54) **BIOPSY NEEDLE GUIDANCE DEVICE FOR STEREOTACTIC BIOPSIES, IMAGING DEVICE COMPRISING SAME AND BIOPSY COLLECTION METHOD USING SAME**

(30) Priority: 06.11.2013 KR 20130134236
(71) Applicant: Rayence Co., Ltd., Hwaseong-si, Gyeonggi-do 445-170 (KR)
(72) Inventor: BAEK, In-Jae, Hwaseong-si Gyeonggi-do 445-170 (KR)
(74) Representative: Markfort, Iris-Anne Lucie
(86) International application number: PCT/KR2014/002150
(87) International publication number: WO 2015/068906

(57) **Abstract**

The present invention relates to a biopsy needle guidance device for enabling stereotactic biopsies with precision and rapidity, an imaging device comprising the same and a biopsy collection method using the same. The biopsy needle guidance device, according to the present invention, includes: a needle guidance device body provided with a needle for tissue collection; and at least one X-ray source provided to the needle guidance device body.

## Description

### BACKGROUND

### 1. Technical Field

The present invention relates to a biopsy needle guidance device and an image obtaining apparatus including the same and a biopsy sampling method using the image obtaining apparatus, and more particularly, to a biopsy needle guidance device which enables stereotatic biopsies precisely and rapidly and an image obtaining apparatus including the same and a biopsy sampling method using the image obtaining apparatus.

### 2. Related Art

X-rays generally refer to electromagnetic waves of a short wavelength corresponding to a wavelength of 0.01 nm to 10 nm and a frequency of 30×10¹⁵ Hz to 30×10¹⁸ Hz. The photographing of X-rays is one of radiographies for projecting and displaying the inside of a specimen with a high penetration force of the X-rays. As well known, X-rays are accompanied by an attenuation phenomenon according to the material, density, and thickness of an object, such as compton scattering and a photoelectric effect, during a process of penetrating the object. Accordingly, the photographing of X-rays includes displaying the inside of a specimen by projecting the inside of the specimen onto a plane based on the amount of attenuation of the X-rays accumulated during the process of transmitting the specimen. To this end, a dedicated X-ray system is used.

Recently, an X-ray imaging technology is grafted on a semiconductor section, and thus is rapidly developed into a digital X-ray imaging technology having various advantages, such as relatively high resolution, a wide dynamic range, and the easy generation of an electrical signal, and the simple processing and storage of data instead of a conventional analog method using a film. The digital-based imaging technology strongly reflects a clinical and environmental need called an early diagnosis of a disease based on the excellent diagnosability of a digital image.

Accordingly, there has been introduced "digital mammography", that is, a breast-dedicated X-ray photographing technology capable of detecting a breast cancer and a lesion and fine calcification for an early diagnosis by representing the internal structure of the breast, that is, a specimen, in a high-resolution image using the biological tissue contrast ability unique to X-rays. Such digital mammography is rapidly spread due to unique characteristics, such as the magnification of an image, a reduction of a photographing number, an increase of resolution, and the minimization of atomic bombing through control of brightness and contrast ratio, in addition to various advantages of the digital X-ray imaging technology.

Furthermore, a biopsy needle module for sampling a tissue having an abnormal portion is installed on a digital mammography apparatus capable of directly checking an abnormal portion (lesion) of the breast through X-ray photographing. Accordingly, the tissue of the abnormal portion can be sampled simultaneously with the checking of the abnormal portion.

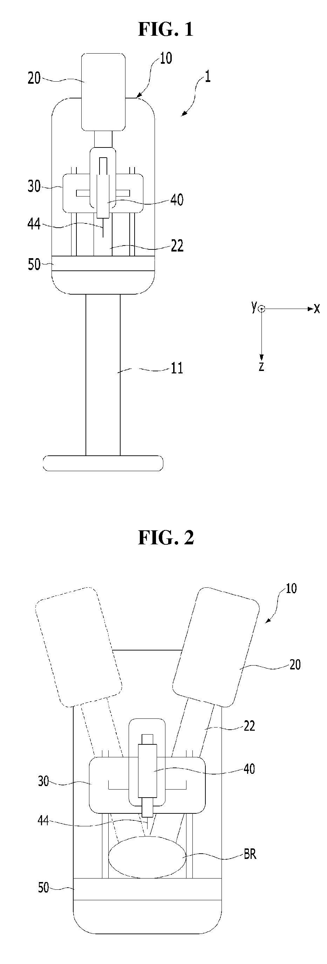

Referring to FIGS. 1 and 2 showing such a conventional mammography apparatus and a device body thereof, the mammography apparatus 1 includes a support column 11 of a vertical pillar shape and a C arm or device body 10 which is installed to move up and down along the support column 11 and has a generally C-frame or similar shape when viewed in an x-axis direction. A generator or X-ray source 20 for radiating X-rays to the bottom of the device body 10 is installed at the top of the device body 10. A detector 50 that faces the X-ray source is installed on the lower side of the device body 10.

The X-ray source 20 is installed on the device body 10 in such a way as to rotate around a y-axis through an X-ray source rotation support unit 22. A needle guiding device holder 30 fixed to the device body 10 is installed between the X-ray source 20 and the detector 50. A biopsy needle guiding device 40 is installed on the needle guiding device holder 30. In this case, the biopsy needle guiding device 40 is installed on the needle guiding device holder 30 in such a way as to move in the x-, y-, z-axis.

In such a mammography apparatus 1, when a testee enters a photographing location in a stand-up or sitting state, the device body 10 moves up and down with respect to the support column 11, and thus the height of the device body 10 is adjusted so that a specimen (BR of FIG. 2), such as the breast of the testee, is placed on a target location on the detector 50. Next, the X-ray source 20 radiates X-rays toward the specimen BR, and the detector 50 placed under the specimen BR obtains images by receiving X-rays passing through the specimen BR.

Next, as shown in FIG. 2, X-ray photographing is additionally performed on the specimen BR on the detector 50 in the state in which the X-ray source rotation support unit 22 has been rotated at a specific angle, for example, 15 degrees to 30 degrees left and right as indicated by a solid line and a dotted line.

A three-dimensional location, that is, the x-, y-, and z-locations of an abnormal portion of the specimen BR (if the abnormal portion is included in the specimen BR), can be obtained using triangulation from three X-ray images of the specimen BR which have been obtained as described above.

Next, the needle guiding device holder 30 moves the biopsy needle guiding device 40 to a corresponding location and moves a needle 44 mounted on the biopsy needle guiding device 40 back and force based on the calculated three-dimensional location data of the abnormal portion, thereby being capable of sampling the tissue of the abnormal portion of the specimen BR.

The mammography apparatus configured as described above, however, requires a rotary driving device for rotating the X-ray source 20 at a specific angle in order to photograph the specimen BR at three different angles. Such a rotary driving device makes complicated an overall structure of this apparatus and increases the size of the apparatus, thereby increasing a unit cost of a product.

In order to obtain the three-dimensional location from the images obtained at three different angles using such a rotary driving device, the X-ray source 20 needs to be rotated at an accurate angle and X-ray photographing needs to be performed in the state in which the X-ray source 20 has been completely stopped after the rotation. Accordingly, it is not so easy to control the rotation of the X-ray source 20 at an accurate angle.

In particular, if the specimen BR is photographed while rotating the X-ray source 20 using the rotary driving device as described above, a lot of time is taken for photographing. Furthermore, a testee and a person to be inspected experience a great sense of fatigue because the specimen BR has to maintain a fixed state during such photographing and until the tissue of the abnormal portion is sampled.

That is, if the specimen BR moves while the specimen BR is photographed, it is difficult to obtain an accurate three-dimensional location of the abnormal portion. Although an accurate three-dimensional location is obtained, the tissue of a required abnormal portion may not be accurately sampled if the specimen BR moves while the biopsy needle guiding device 40 moves to a corresponding location.

### SUMMARY

The present invention has been made to solve the above conventional problems, and an object of the present invention is to provide a biopsy needle guidance device which enables stereotatic biopsies precisely and rapidly through a simple structure and a mammography apparatus including the same and a biopsy sampling method using the mammography apparatus.

A biopsy needle guidance device for achieving the above object in accordance with an aspect of the present invention includes a needle guiding device body on which a tissue sampling needle has been mounted and at least one X-ray source which is installed on the needle guiding device body.

The X-ray source may include one X-ray source movable in a direction crossing the length direction of the tissue sampling needle.

The X-ray source may include two or more X-ray sources spaced apart from each other in a direction crossing the length direction of the tissue sampling needle.

The X-ray sources may be symmetrically disposed left and right at an angular range of 15 degrees to 30 degrees in the same distance based on one point toward the length direction of the tissue sampling needle.

The X-ray source may further include one X-ray source placed in the length direction of the tissue sampling needle.

An image obtaining apparatus in accordance with another aspect of the present invention includes a device X-ray source which radiates X-rays toward a specimen, a detector which detects the X-rays passing through the specimen, a device body which supports the X-ray source and the detector, a needle guiding device body which is installed on the device body between the device X-ray source and the detector and on which a tissue sampling needle has been mounted, and a biopsy needle guiding device which includes at least one X-ray source installed on the needle guiding device body.

The biopsy needle guiding device body may be detachably configured in the device body.

A biopsy sampling method in accordance with yet another aspect of the present invention may include the steps of obtaining images passing through a specimen and determining whether the specimen is normal or not using an image obtaining apparatus including a device X-ray source and a detector which are disposed with the specimen interposed between the device X-ray source and the detector and a biopsy needle guiding device which is disposed between the specimen and the device X-ray source and includes a tissue sampling needle and at least one X-ray source and calculating a three-dimensional location of an abnormal portion of the specimen using the needle X-ray source and the detector and sampling biopsies of the abnormal portion using the tissue sampling needle.

The biopsy needle guidance device configured as described above and the image obtaining apparatus including the same and the biopsy sampling method using the image obtaining apparatus according to the present invention can reduce an X-ray photographing and tissue sampling time because a three-dimensional location of an abnormal portion can be detected rapidly and accurately and the tissue of the abnormal portion can be sampled.

A mammography apparatus, that is, the image obtaining apparatus according to the present invention, can facilitate the maintenance and repair of the apparatus because it is implemented to have a simple configuration not having a movable part, can increase lifespan, and can reduce the size of the apparatus. For this reason, a manufacturing unit cost for the image obtaining apparatus can also be reduced.

Furthermore, in accordance with the biopsy needle guidance device and the image obtaining apparatus including the same and the biopsy sampling method using the image obtaining apparatus according to the present invention, a degree of fatigue of a testee and a person to be inspected can be reduced because an abnormal portion is photographed rapidly and accurately and the tissue of the abnormal portion is sampled.

Furthermore, the range in which existing equipment is used can be widened because stereotatic biopsies are enabled by mounting the biopsy needle guidance device according to the present invention on an existing image obtaining apparatus incapable of stereotatic biopsies.

### BRIEF DESCRIPTION OF THE DRAWINGS

Other objects, characteristics, and advantages of the present invention may become evident from the following description of preferred embodiments given in connection with the following accompanying drawings.
FIG. 1 is a front view of a conventional mammography apparatus.
FIG. 2 is a front view of the device body of the mammography apparatus of FIG. 1 and is a diagram illustrating a stereotatic biopsy process.
FIG. 3 is a front view of a mammography apparatus according to the present invention.
FIG. 4 is a front view of a biopsy needle guidance device mounted on the mammography apparatus of FIG. 3.
FIG. 5 is a front view of the device body of the mammography apparatus according to the present invention and is a diagram illustrating a stereotatic biopsy process.
FIG. 6 is a front view of another embodiment of the biopsy needle guidance device mounted on the mammography apparatus of FIG. 3.

### DETAILED DESCRIPTION

Hereinafter, embodiments of the present invention are described in detail with reference to the accompanying drawings. The following embodiments are provided as examples in order to sufficiently deliver the spirit of the present invention to those skilled in the art. Accordingly, the present invention is not limited to the following embodiments, but may be embodied in different forms. Furthermore, in the drawings, the width, length, thickness, etc. of each element may have been enlarged for convenience. The same reference numbers are used throughout the specification to refer to the same elements.

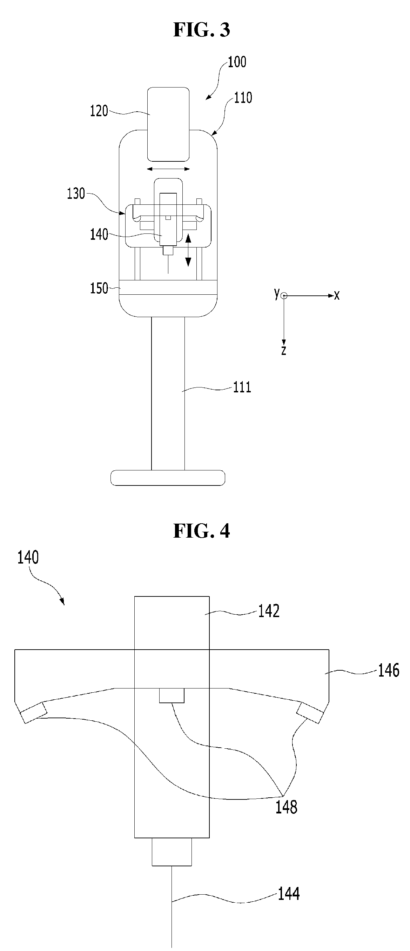

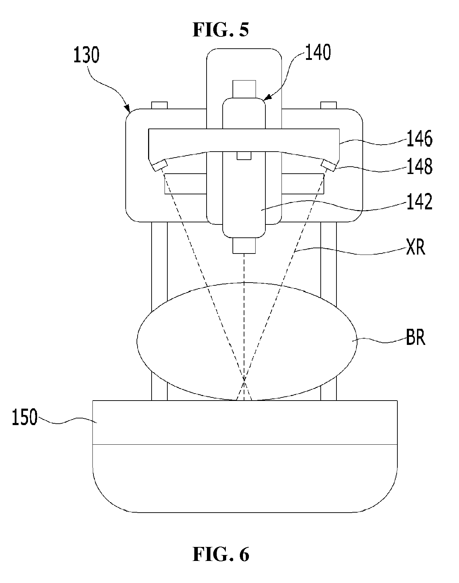

FIG. 3 is a front view of a mammography apparatus according to the present invention. FIG. 4 is a front view of a biopsy needle guidance device mounted on the mammography apparatus of FIG. 3. FIG. 5 is a front view of the device body of the mammography apparatus according to the present invention and is a diagram illustrating a stereotatic biopsy process.

First, referring to FIG. 3, the mammography apparatus 100, that is, an example of an image obtaining apparatus according to the present invention, includes a device body 110 including basic elements for X-ray photographing and a support column 111 which guides the device body 110 so that it is vertically movable. The support column 111 has a vertical pillar shape and enables the device body 110 to move up and down along the support column 111.

The device body 110 has an arc whose upper and lower ends face each other, and may be indicative of a generally C-frame or similar shape when viewed in an x-direction. A generator or device X-ray source 120 for radiating X-rays to the bottom of the device body 110 is installed at the top of the device body 110. A detector 150 for receiving X-rays is installed on the lower side of the device body 110 in such a way as to face the device X-ray source 120.

A needle guiding device holder 130 fixed to the device body 110 is installed between the device X-ray source 120 and the detector 150. A biopsy needle guiding device 140 is detachably installed on the needle guiding device holder 130.

The device X-ray source 120 mounted on the top of the device body 110 is a device for generating X-rays by colliding electrons having high kinetic energy against a metal target, and preferably includes an optical system, such as a collimator for controlling the radiation direction or radiation area of X-rays.

The detector 150 mounted on the lower side of the device body 110 is basically means for obtaining images by receiving the X-rays passing through a specimen BR, such as the breast, and may also function as a specimen support unit for supporting the specimen BR when the specimen BR is placed on the detector 150. That is, after the specimen BR is placed on the detector 150, the specimen BR is photographed by the device X-ray source 120 and the detector 150.

In this case, although not shown, in order to prevent an abnormal portion within the specimen BR from being covered by a mammary gland tissue, it is preferred that a compression pad for pressurizing the specimen BR placed on the detector 150 may be included between the device X-ray source 120 and detector 150 of the device body 110. The compression pad may pressurize the specimen BR when X-ray photographing is performed on the specimen BR.

Furthermore, the detector 150 may generate an electrical signal for each location which is proportional to the amount of incident X-rays, may read the electrical signals and location information, and may obtain X-ray images of the specimen BR by processing the read electrical signals and location information using an image processing algorithm. In this case, the contents of a common technology, such as a direct conversion method for directly obtaining an electrical signal from X-rays without a separate middle step or an indirect conversion method for converting X-rays into a visible ray and indirectly obtaining an electrical signal from the visible ray, may be widely applied to the detector 150 depending on a method for converting X-rays.

The needle guiding device holder 130 is an element for retaining and supporting the biopsy needle guiding device 140 so that the biopsy needle guiding device 140, that is, a biopsy device, is placed between the device X-ray source 120 and the detector 150. The needle guiding device holder 130 may be fixed and installed on the device body 110 or may be installed on the device body 110 in such a way as to be movable with respect to a z-axis, if necessary. It is preferred that the biopsy needle guiding device 140 may be retained and supported by the needle guiding device holder 130 in such a way as to be detachably attached. The needle guiding device holder 130 includes an x-axis and y-axis stage for enabling the biopsy needle guiding device 140 to move in the x-axis and y-axis directions, and may selectively include a z-axis stage for enabling the biopsy needle guiding device 140 to move in the z-axis direction.

The biopsy needle guiding device 140 is a biopsy device equipped with a needle which is inserted into a specimen and samples the tissue of the specimen. The biopsy needle guiding device 140 is detachably installed on the needle guiding device holder 130. As shown in FIG. 4, the biopsy needle guiding device 140 according to the present invention includes a needle guiding device body 142 of a shape lengthily extended in a vertical direction, a tissue sampling needle 144 mounted on the bottom of the needle guiding device body 142, an X-ray source mounting unit 146 horizontally extended on one side of the needle guiding device body 142, and three needle X-ray sources 148 mounted on the X-ray source mounting unit 146. Furthermore, the three needle X-ray sources 148 are spaced apart from each other at a specific interval and installed in the length direction of the X-ray source mounting unit 146.

Meanwhile, a common X-ray source includes a field emission cathode for emitting electrons and a target metal unit, that is, an anode for generating X-rays through a collision with the emitted electrons. The field emission cathode is an element for emitting electrons by applying an electric field in a vacuum environment. A filament, etc. was conventionally used as the field emission cathode. However, a nano emitter, such as a carbon nanotube, is recently used with the development of a nano technology. An X-ray source using a carbon nanotube solves excessive power consumption attributable to the heating of a filament cathode and also improves an electron beam focusing limit through a further reduced size, compared to the conventional X-ray source using a filament cathode. A detailed configuration and operation of such an X-ray source have been widely known in the technical field to which the present invention pertains, and a detailed description thereof is omitted.

The three needle X-ray sources 148 can be easily spaced apart from each other at specific interval and installed because a carbon nanotube X-ray source having a reduced size as described above is used in the biopsy needle guiding device 140 according to the present invention.

It is preferred that the needle X-ray source 148 (hereinafter referred to as a "center needle X-ray source 148") that belongs to the three needle X-ray sources 148 and that is placed at the center is placed on the extension line of the tissue sampling needle 144 in the length direction thereof. It is preferred that the remaining two needle X-ray sources 148 are spaced apart from each other at a specific interval and placed on the left and right of the central needle X-ray source 148. Furthermore, when the biopsy needle guiding device 140 is placed in a height of a standby location, assuming that a point vertically projected on a surface of the detector 150 from the central needle X-ray source 148 is a reference point, the three needle X-ray sources 148 are installed toward the reference point in the same distance from the reference point. That is, the three needle X-ray sources 148 are arranged toward the reference point along an arc for the reference point. In this case, it is preferred that the three needle X-ray sources 148 are spaced apart from each other at an angle of 15 degrees to 30 degrees using the reference point as the center line.

A method for performing X-ray photographing on the specimen BR, such as the breast of a testee, and then sampling the tissue of an abnormal portion using the mammography apparatus 100 configured as described above according to the present invention is described below.

First, when the testee in a stand-up or sitting state is placed at the photographing location of the mammography apparatus 100, the device body 110 moves up and down along the support column 111 and is thus positioned so that the specimen BR of the testee is placed on the detector 150.

Next, the device X-ray source 120 radiates X-rays toward the specimen BR. The detector 150 placed beneath the specimen BR receives the X-rays passing through the specimen BR. The detector 150 may generate an electrical signal for each location which is proportional to the amount of received incident X-rays, may read the electrical signals and location information, and may obtain X-ray images of the specimen BR by processing the read electrical signals and location information using an image processing algorithm. If an abnormal portion within the specimen BR is detected, the detector 150 obtains a two-dimensional location within the x-y plane of the abnormal portion from an image obtained by the device X-ray source 120.

Next, the biopsy needle guiding device 140 is moved to an approximate location of the obtained abnormal portion by driving the x-axis and y-axis stage of the needle guiding device holder 130. That is, the biopsy needle guiding device 140 is approximately placed over the abnormal portion. Next, the three needle X-ray sources 148 mounted on the X-ray source mounting unit 146 of the biopsy needle guiding device 140 sequentially (or simultaneously) radiate X-rays XR toward the specimen BR, more specifically, the abnormal portion. The detector 150 placed beneath the specimen BR obtains images by receiving the X-rays XR passing through the specimen BR.

A three-dimensional location of the abnormal portion, that is, x-, y-, z-locations, may be obtained using triangulation from three X-ray images of the specimen BR near the abnormal portion, which have been obtained as described above. Next, the biopsy needle guiding device 140 is accurately placed over the abnormal portion by precisely driving the x-axis and y-axis stage of the needle guiding device holder 130 based on the x- and y-location data of the abnormal portion obtained by the three needle X-ray sources 148. That is, the biopsy needle guiding device 140 is positioned at an accurate location. Finally, the biopsy needle guiding device 140 or the needle guiding device holder 130 according to circumstances moves the tissue sampling needle 144 back and forth in the z-direction based on the z-location data of the abnormal portion obtained by the three needle X-ray sources 148 and samples the tissue of the abnormal portion.

As described above, after the biopsy needle guiding device 140 is moved to the approximate location of the abnormal portion obtained by the device X-ray source 120, the three needle X-ray sources 148 of the biopsy needle guiding device 140 precisely photograph the three-dimensional location of the abnormal portion again. Accordingly, there is no obstacle to detecting a three-dimensional location of the tissue of the abnormal portion although there is some movement in the specimen BR until the specimen BR is photographed again by the needle X-ray source 148 of the biopsy needle guiding device 140 after the specimen BR is photographed by the device X-ray source 120. Accordingly, a degree of fatigue of a testee and a person to be inspected during photographing can be reduced.

Furthermore, the mammography apparatus 100 according to the present invention is implemented using a simple configuration without a movable part for rotating the device X-ray source for stereotatic biopsies. Accordingly, the maintenance and repair of the apparatus are facilitated, lifespan is increased, the entire mammography apparatus can be reduced in size, and a manufacturing unit cost for the apparatus can also be reduced.

In the aforementioned embodiment, the three needle X-ray sources 148 have been installed on the biopsy needle guiding device 140, but an implementation example of the present invention may include that only two needle X-ray sources are installed on the biopsy needle guiding device 140 and the device X-ray source 120 installed on the device body 110 of the mammography apparatus 100 is used. That is, a three-dimensional location of an abnormal portion of the specimen BR may be measured using three X-ray sources, including two needle X-ray sources installed on the biopsy needle guiding device 140 and one device X-ray source 120 installed on the device body 110.

In this case, when the biopsy needle guiding device 140 is moved in order to measure the three-dimensional location of the abnormal portion of the specimen BR, it is to be noted that the abnormal portion, one of the two needle X-ray sources, and the device X-ray source 120 should not be placed on the same line. If this is taken into consideration, it is preferred that the needle X-ray source 148 that belongs to the three needle X-ray sources 148 installed on the biopsy needle guiding device 140 shown in FIG. 4 and that is placed at the center is omitted.

In particular, when the biopsy needle guiding device 140 is moved within the mammography apparatus 100, the needle X-ray sources move along with the biopsy needle guiding device 140, but the device X-ray source 120 is fixed. Accordingly, when X-ray photographing for measuring a three-dimensional location of an abnormal portion using the two needle X-ray sources and the device X-ray source 120 is performed, a location relation between the X-ray sources needs to be taken into consideration. Furthermore, correction for the distance from the abnormal portion of the specimen BR to the needle X-ray source and the distance from the abnormal portion of the specimen BR to the device X-ray source 120 needs to be taken into consideration because the distances are different.

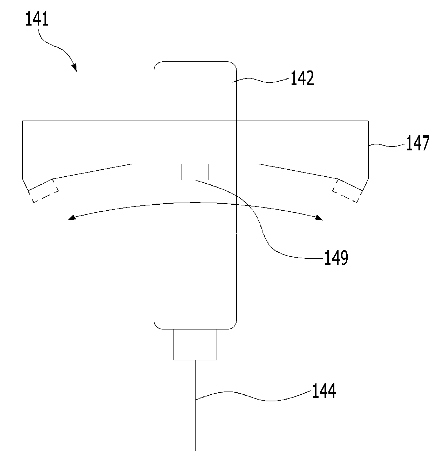

Meanwhile, the present invention may be implemented by installing a single needle X-ray source in the biopsy needle guiding device 140 and moving the single needle X-ray source. For example, a biopsy needle guiding device 141 according to another embodiment of the present invention shown in FIG. 6 includes the needle guiding device body 142 of a shape lengthily extended in a vertical direction, the tissue sampling needle 144 mounted on the bottom of the needle guiding device body 142, an X-ray source mounting unit 147 extended in a horizontal direction on one side of the needle guiding device body 142, and a single needle X-ray source 149 mounted on the X-ray source mounting unit 147 in such a way as to move in the length direction of the X-ray source mounting unit 147. The needle X-ray source 149 is configured to be movable in the direction indicated by an arrow in FIG. 6 and to perform X-ray photographing on the specimen BR at three locations indicated by a solid line and dotted lines, for example.

In this case, it is preferred that the X-ray source mounting unit 147 on which the needle X-ray source 149 is movably mounted has an arc shape so that the needle X-ray source 149 moves toward the reference point described with reference to FIG. 4 along the X-ray source mounting unit 147 while having the same distance to the reference point. Meanwhile, it is preferred that the three locations indicated by the solid line and dotted lines in FIG. 6 are spaced apart from each other at an angle of 15 degrees to 30 degrees using the reference point as a central point with respect to a location indicated by the solid line at the center.

As described above, the needle guidance device 140 or 141 on which one or more small-sized X-ray sources according to the present invention have been mounted is installed on an existing mammography apparatus. Accordingly, the range in which existing equipment is used can be widened because stereotatic biopsies are made possible even in the existing mammography apparatus incapable of stereotatic biopsies. That is, a competition with an expensive high-end product capable of stereotatic biopsies is possible because the biopsy needle guiding device 140 or 141 according to the present invention is mounted on a low-priced screening-dedicated product.

While some embodiments of the present invention have been described, those skilled in the art to which the present invention pertains may change and modify the present invention in various ways without departing from the essential characteristic of the present invention. Accordingly, the aforementioned embodiments should not be construed as limiting the technical spirit of the present invention, but should be construed as illustrating the technical spirit of the present invention for better understanding. The scope of the present invention is not restricted by such embodiments, and the scope of the present invention should be interpreted based on the following appended claims. Accordingly, the present invention should be construed as covering all modifications or variations derived from the meaning and scope of the appended claims and their equivalents.

## Claims

1. A biopsy needle guiding device, comprising:
a needle guiding device body on which a tissue sampling needle has been mounted, and
at least one X-ray source which is installed on the needle guiding device body.

2. The biopsy needle guiding device of claim 1, wherein the X-ray source comprises one X-ray source movable in a direction crossing a length direction of the tissue sampling needle.

3. The biopsy needle guiding device of claim 1, wherein the X-ray source comprises two or more X-ray sources spaced apart from each other in a direction crossing a length direction of the tissue sampling needle.

4. The biopsy needle guiding device of claim 3, wherein the X-ray sources are symmetrically disposed left and right at an angular range of 15 degrees to 30 degrees in an identical distance based on one point toward the length direction of the tissue sampling needle.

5. The biopsy needle guiding device of claim 3 or 4, wherein the X-ray source further comprises one X-ray source placed in the length direction of the tissue sampling needle.

6. An image obtaining apparatus, comprising:
a device X-ray source which radiates X-rays toward a specimen,
a detector which detects the X-rays passing through the specimen,
a device body which supports the X-ray source and the detector, and
a needle guiding device body which is installed on the device body between the device X-ray source and the detector and on which a tissue sampling needle has been mounted, and
a biopsy needle guiding device which comprises at least one X-ray source installed on the needle guiding device body.

7. The image obtaining apparatus of claim 6, wherein the biopsy needle guiding device body is detachably configured in the device body.

8. A biopsy sampling method, comprising steps of:
obtaining images passing through a specimen and determining whether the specimen is normal or not using an image obtaining apparatus comprising a device X-ray source and a detector which are disposed with the specimen interposed between the device X-ray source and the detector and a biopsy needle guiding device which is disposed between the specimen and the device X-ray source and comprises a tissue sampling needle and at least one X-ray source; and
calculating a three-dimensional location of an abnormal portion of the specimen using the needle X-ray source and the detector and sampling biopsies of the abnormal portion using the tissue sampling needle.
